# EUROPÄISCHE PATENTSCHRIFT

(11) **EP 3 071 282 B1**
(45) Veröffentlichungstag und Bekanntmachung des Hinweises auf die Patenterteilung: **21.04.2021**
(21) Anmeldenummer: 13840194.8
(22) Anmeldetag: 22.10.2013
(51) Int. Cl.: A61M 25/06, A61M 25/00

(54) **KATHETERPUNKTIONSBESTECK**
CATHETER PUNCTURE DEVICE
ENSEMBLE DE PONCTION À CATHÉTER

(43) Veröffentlichungstag der Anmeldung: 28.09.2016
(73) Patentinhaber: Tietze, Bernd, 91325 Adelsdorf (DE)
(72) Erfinder: Tietze, Bernd, 91325 Adelsdorf (DE)
(74) Vertreter: Kodron, Felix
(86) Internationale Anmeldenummer: PCT/IB2013/059536
(87) Internationale Veröffentlichungsnummer: WO 2015/059517

(56) Entgegenhaltungen:
- WO-A1-02/053045
- WO-A1-2010/111283
- DE-A1- 2 340 534
- DE-U1-202013 102 968
- US-A1- 2012 197 200
- US-B1- 6 921 391

## Beschreibung

Die Erfindung betrifft ein Katheterpunktionsbesteck gemäß dem Oberbegriff des Anspruchs 1.

Ist es zur Versorgung eines Patienten in stationärer Behandlung notwendig, einen Katheter zu legen, so erfolgt dies durch das medizinische Personal in der Regel nach der sogenannten "Seldinger"-Methode. Dabei wird in einem ersten Schritt das Gefäß (häufig eine Arterie oder Vene), in das der Katheter einzuführen ist, mit einer hohlen Punktionsnadel punktiert. Danach wird durch die hohle Punktionsnadel in das Gefäß ein Führungsdraht eingeschoben. Die Hohlnadel wird anschließend über den innen liegenden Führungsdraht wieder zurückgezogen, so dass der Führungsdraht weiter in das punktierte Gefäß vorgeschoben werden kann um dieses unter Verwendung eines über den Führungsdraht geschobenen Dilatators zunächst etwas aufzuweiten. Nach Entfernen des Dilatators wird der eigentliche Katheter über den liegenden Führungsdraht in das Gefäß eingeschoben und in die gewünschte Endstellung verschoben. Als letzter Schritt wird der Führungsdraht durch den Katheter wieder vorsichtig herausgezogen.

Diese Methode zur Punktion von Blutgefäßen nach Seldinger zum Zweck der Katheterisierung ist zwar eine Standardmethode, aber zum Legen des Katheters sind hierbei eine Vielzahl verschiedener Handgriffe und Instrumente erforderlich, die eine große Erfahrung in diesem Bereich voraussetzen. Zudem besteht aufgrund des mehrfachen Einschiebens und Herausziehens verschiedener Gegenstände die Gefahr, dass es zu Verunreinigungen von Gegenstände oder Personen durch austretendes Blut kommt. Schließlich ist auch die Gefahr einer Luftembolie gegeben, wenn nämlich ein leichter Unterdruck im punktierten Gefäß herrscht. Es sind daher Katheterpunktionsbestecke als Hilfsmittel zur Katheterisierung bekannt, die eine vereinfachte Handhabung bewirken sollen.

Aus der DE 101 00 102 ist ein Katheterpunktionsbesteck bekannt, welches das Legen eines Katheters vereinfacht, indem es die für eine Punktion und das Legen eines Katheters erforderlichen Utensilien in einem aus mehreren Teilen zusammengeführten System bereitstellt und dabei ohne einen Führungsdraht oder zusätzliche Dilatationsgeräte auskommt.

Das dort offenbarte Katheterpunktionsbesteck umfasst dabei ein rohrartiges Gehäuse mit einem länglichen Gehäuseabschnitt, der in einen Verlängerungsabschnitt übergeht, und von dem unter einem Winkel ein Abzweigabschnitt abzweigt. Der Gehäuseabschnitt und der Abzweigabschnitt weisen eine durchgehende seitliche Öffnung auf, die von einem sich längs des Abzweigabschnitts und des Gehäuseabschnitts erstreckenden, flexiblen Mantel verschlossen wird, in den ein Katheter einschiebbar ist. Die sich längs des länglichen Gehäuseabschnitts und des Verlängerungsabschnitts erstreckende Punktionsnadel tritt bei der Punktion des Gefäßes aus der Spitze des Gehäuses aus und wird nach erfolgter Punktion in das Gehäuse bis hinter die Abzweigstelle des Abzweigabschnitts zurückgezogen. Am hinteren Ende der Punktionsnadel befindet sich ein Blutauffangbehälter.

Anschließend wird der Katheter über den Abzweigabschnitt und den Gehäuseabschnitt aus dem Gehäuse durch den flexiblen Mantel in das punktierte Gefäß eingeführt bis zur gewünschten Tiefe. Erst dann wird das Katheterpunktionsbesteck aus dem punktierten Gefäß des Patienten herausgezogen. Zuletzt wird der flexible Mantel aus dem Gehäuse herausgezogen bzw. über einen seitlichen Längsschlitz vom Katheter abgeschält. Insgesamt liegen also bei dem offenbarten Katheterpunktionsbesteck nach der DE 101 00 102 nach erfolgter Punktion neben dem gelegten Katheter zwei Teile vor, zum einen das Gehäuse mit der Punktionsnadel und dem Blutauffangbehälter, zum anderen der abgelöste Mantel.

Aus der Veröffentlichung US 2012/197200 A1 ist ein Venen-Zugangskatheter offenbart der eine Nadel, einen Führungsdraht und einen Aktuator kombiniert, wobei die Nadel koaxial über dem Führungsdraht angeordnet ist und der Katheter koaxial über der Nadel angeordnet ist. Eine Nabe an einem proximalen Ende des Zugangskatheters umfasst ein Abstreifelement zum Entfernen von Blut von Nadel und Führungsdraht, wenn sie entfernt werden, und eine Seitenöffnung, um den Anschluss von Flüssigkeiten zu ermöglichen, nachdem der Zugangskatheter platziert wurde.

Die Druckschrift WO 2010/111283 A1 offenbart eine universelle Greiffläche an einer intravenösen Katheteranordnung. Die universelle Greiffläche ermöglicht hierbei dem Anwender, die Katheteranordnung in einer gewünschten Greifkonfiguration ergreifen zu können, um das Gleichgewicht und die Kontrolle der Katheteranordnung während des Einführens des Katheters zu verbessern.

Eine weitere Druckschrift US 6 921 391 B1 offenbart eine Infusionsvorrichtung zur Verwendung mit einem intravenösen Katheter, der an eine Primärfluidversorgung angeschlossen ist. Die Infusionsvorrichtung ist hierbei lösbar mit einer Y-Anschlussstelle verbindbar, um eine Sekundärfluidversorgung mit dem Katheter zu verbinden und umfasst eine einziehbare Nadel, die nach Gebrauch zurückgezogen werden kann.

Auch aus der Veröffentlichung DE 23 40 534 A1 ist eine Verlegehilfe für einen Venenkatheter mit einem Führungsrohr bekannt, das durch eine Verbindungsstelle vom Y-Typ mit einem zweiten Führungsrohr für eine Punktionsnadel verbunden ist, um einen gemeinsamen Führungsabschnitt zu ergeben. Das Führungsrohr wird in die Öffnung einer Vene eingeführt und weist einen inneren Kanal auf, der zur Aufnahme des Katheterrohrs dimensioniert ist, und der längs teilbar ist, um von dem eingeführten Katheter entfernt zu werden.

Schließlich ist aus der Druckschrift DE 20 2013 102 968 U1 ein Katheterpunktionsbesteck bekannt umfassend ein rohrartiges Gehäuse mit einem länglichen Gehäuseabschnitt, der in einen Verlängerungsabschnitt zur Führung der Punktionsnadel mit Blutauffangbehälter, Anschlag und Sperrelement übergeht, und von dem unter einem Winkel ein Abzweigabschnitt abzweigt, über welchen ein Katheter in den länglichen Gehäuseabschnitt einschiebbar ist, wobei nach erfolgter Punktion eine Rückführung der Punktionsnadel hinter die Abzweigstelle des Abzweigabschnitts erfolgt und entlang des Abzweigabschnitts bis zur Spitze des länglichen Gehäuseabschnitts zumindest eine Sollbruchstelle verläuft, mithilfe derer das rohrartige Gehäuse des Katheterpunktionsbestecks, nachdem der Katheter hindurch geschoben wurde, aufgebrochen werden kann, um das Gehäuse vom Katheter zu entfernen.

Es sind neben diesem geschilderten Stand der Technik noch weitere Methoden der Katheterlegung nach modifizierter Seldingertechnik bekannt, wobei einige der stets mehrteiligen Katheterpunktionsbestecke mit der sog. "Peel-Away"-Technik arbeiten, bei welcher sich die Einführhülsen nach erfolgter Katheterplatzierung im punktierten Gefäß an einer "Peel-Away-Schleuse" in zwei Teile teilen und aus dem Gefäß ohne eine Bewegung des Katheters herausgezogen werden können.

Es ist vor diesem Hintergrund die Aufgabe der vorliegenden Erfindung, ein Katheterpunktionsbesteck zu schaffen, welches das Legen eines Katheters konstruktiv weiter vereinfacht und gleichzeitig dessen Handhabbarkeit verbessert, wobei die Zahl der benötigten einzelnen Bauteile des Katheterpunktionsbestecks reduziert wird.

Erreicht wird dies durch ein Katheterpunktionsbesteck mit den kennzeichnenden Merkmalen des Anspruch 1.

Die Unteransprüche haben vorteilhafte Ausgestaltungen der Erfindung zum Gegenstand.

Das erfindungsgemäße Katheterpunktionsbesteck stellt ein geschlossenes System dar, welches alle für die Punktion notwendige Bauteile in eine Vorrichtung vereint und zur Verfügung stellt und gleichzeitig ermöglicht, zum einen die Handhabbarkeit des Katheterpunktionsbestecks durch Ablösen der nicht mehr benötigten baulichen Komponenten nach der Punktion zu verbessern, und zum anderen den Katheter nach erfolgter Einführung in das punktierte Gefäß mit einem Handgriff aus dem verbliebenen baulichen Komponenten des Katheterpunktionsbestecks zu lösen und freizulegen.

Kern der erfinderischen Idee ist es, das Legen des Katheters dadurch zu vereinfachen, dass nach der Punktierung des Gefäßes und dem Einführen des Katheters in die Einführhülse des Katheterpunktionsbestecks eine Teilung des Katheterpunktionsbestecks in die baulichen Komponenten erfolgen kann, die nicht mehr benötigt werden und solche, die zum Abschließenden Einführen des Katheters in das punktierte Gefäße noch erforderlich sind.

Erreicht wird dies durch die bauliche Komponente eines Sicherheitsgehäuses, welches als zentrales verbindendes Bauteil die konstruktiv gepaarten Elemente der Einführhülse mit seitlich einschiebbarem Katheter einerseits mit der Punktionsnadel und dem Blutauffangbehälter andererseits verbindet, wobei das Sicherheitsgehäuse konstruktiv letzter Baugruppe zuzuordnen ist, da es neben seiner Funktion als Bindeglied auch der Aufnahme und Sicherung des Kopfes der Punktionsnadel dient, wenn diese aus der Einführhülse nach erfolgter Punktion zurückgeführt wird.

Das Sicherheitsgehäuse ist hierbei in der Ausgangsposition des Katheterpunktionsbestecks zudem Angriffspunkt für die Sicherung des Blutauffangbehälters mit daran angesetzter Punktionsnadel. Das heißt, um die Punktionsnadel beim Punktieren des Gefäßes gegen eine Längsverschiebung in der Einführhülse zu sichern, ist diese oder ist der Blutauffangbehälter mit aufgesetzter Punktionsnadel mittels einer lösbaren Verbindung am Sicherheitsgehäuse befestigt.

Diese lösbare Verbindung zwischen Blutauffangbehälter oder Punktionsnadel und dem Sicherheitsgehäuse kann bspw. durch eine Schraubverbindung erfolgen. Es hat sich allerdings als besonders vorteilhaft erwiesen, eine leicht lösbare Schnappverbindung vorzusehen, die durch das Anheben einen Arretierungsarmes gelöst werden kann, da so das medizinische Personal schnell und ohne Mühe die Lösung der Verbindung herbeiführen und die Punktionsnadel mit Blutauffangbehälter aus der Einführhülse zurückziehen kann, bis der Punktionsnadelkopf im Sicherheitsgehäuse anschlägt und derart einrastet, dass die Punktionsnadel nicht mehr aus dem Sicherheitsgehäuse verschoben werden kann.

An dieser Stelle ist nun der besondere Vorteil des erfindungsgemäßen Sicherheitsgehäuses zu erkennen. Sind nämlich die Punktionsnadel mit Blutauffangbehälter aus der Einführhülse zurückgeführt worden, kann der Katheter durch das Katheterpunktionsbesteck geschoben werden. Hierbei hat es sich allerdings als hinderlich erwiesen, dass im Stand der Technik die weit nach hinten ragende Punktionsnadel mit Blutauffangbehälter weiterhin mit dem Katheterpunktionsbesteck verbunden sind und so dessen Handhabbarkeit beim Vorschieben des Katheters sowie beim abschließenden Trennen des gelegten Katheters vom Katheterpunktionsbesteck erschweren.

Das Sicherheitsgehäuse ist daher so ausgebildet, dass es von der Einführhülse mit Katheter nach Rückführung der Punktionsnadel abgenommen werden kann. Die Verbindung zwischen Einführhülse und Sicherheitsgehäuse ist demnach lösbar ausgebildet, wobei es sich als vorteilhaft gezeigt hat, analog zur zuvor beschriebenen Verbindung eine leicht lösbare Schnappverbindung vorzusehen, die durch einfaches Anheben eines Arretierungsarmes gelöst werden kann, da so das medizinische Personal schnell und ohne Mühe die Lösung der Verbindung herbeiführen kann. Auch andere alternative leicht lösbare Verbindungsmittel sind hierfür denkbar.

Auf diese Weise wird erreicht, dass sobald der Katheter in die Einführhülse zum Blutgefäß vorgeschoben wurde und somit das aufsteigende Blut aus dem Blutgefäß im Katheter kanalisiert ist, das Sicherheitsgehäuse entriegelt und von der Einführhülse mit Katheter abgenommen werden kann. Im Sicherheitsgehäuse sind dabei die Punktionsnadel und der mit dieser verbundene Blutauffangbehälter arretiert, wodurch diese gemeinsam mit dem Sicherheitsgehäuse vollständig vom Katheterpunktionsbesteck entfernt werden können und eine Verletzung des medizinischen Personals an der im Sicherheitsgehäuse fixierten Punktionsnadel ausgeschlossen ist. Das Katheterpunktionsbesteck besteht daher nach diesem Schritt nur noch aus der Einführhülse mit darin verlaufenden Katheter. Entsprechend unproblematisch ist die Handhabung dieser verbleibenden Elemente des Katheterpunktionsbestecks.

Im abschließenden Arbeitsschritt muss nun lediglich der Katheter durch die Einführhülse in seine Endposition im Blutgefäß vorgeschoben werden. Gleichzeitig kann die Einführhülse wieder aus dem Blutgefäß zurückgeführt werden. Durch zumindest eine Sollbruchstelle, bspw. eine Peel-Away-Sollbruchstelle, entlang der Einführhülse kann diese nun geöffnet und vom Katheter abgenommen werden, der so abschließend gelegt worden ist.

Durch die rückwärtig herausragende Punktionsnadel sowie das an dieser aufgesetzte Blutauffanggefäß entsteht im Stand der Technik ein sehr hinderlicher Fortsatz am Katheterpunktionsbesteck, der gerade im sensiblen Moment des Einschiebens des Katheters die Handhabbarkeit stark einschränkt und so falsche Anwendungen verursacht bzw. einige Geschicklichkeit und Übung voraussetzt. Dieser Mangel ist durch das erfindungsgemäße ablösbare Sicherheitsgehäuse vollständig ausgeräumt.

Eine vorteilhafte Bauform des erfindungsgemäße Katheterpunktionsbesteck-Gehäuses ist nur noch einteilig ausgebildet, wobei der längliche, rohrförmig geschlossene Gehäuseabschnitt, der als Einführhülse bezeichnet wird, sowohl eine mit ihrer Spitze aus dem länglichen Gehäuseabschnitt austretende, sich längs des länglichen Gehäuseabschnitts und des Verlängerungsabschnitts erstreckende Punktionsnadel führt, als auch gleichzeitig den Führungskanal für den Katheter bildet, der über eine Abzweigstelle in diesen Führungskanal einführbar ist. Nach erfolgter Punktion des Gefäßes durch die Punktionsnadel und Rückführung der Punktionsnadel bis hinter die Abzweigstelle des Abzweigabschnitts kann so der Katheter über den Abzweigabschnitt in den länglichen Gehäuseabschnitt eingeführt werden, wobei entlang des Abzweigabschnitts bis zur Spitze des einteiligen länglichen Gehäuseabschnitts zumindest eine Sollbruchstelle angeordnet ist, die eine Öffnung des Gehäuses ermöglicht, so dass der Katheter nach erfolgter Öffnung freigelegt ist und das Katheterpunktionsbesteck entfernt werden kann.

So umfasst das erfindungsgemäße Katheterpunktionsbesteck in einer einzigen Einführhülse zunächst die Punktionsnadel, die an einem Ende aus dem länglichen Gehäuseabschnitt herausgeschoben wird und das Gefäß punktiert. Dabei wird auch das Ende die Einführhülse selbst ein wenig in das punktierte Gefäß mit eingeschoben. Wurde das Blutgefäß punktiert, tritt Blut in die Punktionsnadel ein, welches in einem am anderen Ende der Punktionsnadel angeordneten, vorzugsweise durchsichtigen Blutauffangbehälter aufgenommen wird.

Die Punktionsnadel wird so weit zurückgezogen, bis die Spitze hinter der Abzweigstelle des Abzweigabschnitts des Gehäuses bzw. der Einführhülse im Sicherheitsgehäuse zu liegen kommt und ein an der Punktionsnadel angeordneter Anschlag an einem entsprechenden Gegenlager im Sicherheitsgehäuse anschlägt, damit die Punktionsnadel nicht vollständig aus dem Katheterpunktionsbesteck herausgezogen werden kann. Um ein erneutes Einschieben der Punktionsnadel ebenfalls sicher zu vermeiden, sichert ein Sperrelement die Punktionsnadel, so dass deren mit Blut verunreinigte Spitze sicher im Sicherheitsgehäuse zu liegen kommt und keine Verletzungsgefahr für das medizinische Personal oder anderen Personen darstellt.

Anschließend wird der Katheter über den Abzweigabschnitt durch das Gehäuse in das punktierte Gefäß eingeführt und soweit in das Gefäß hineingeschoben wie gewünscht. Am Katheter können bei einer vorteilhaften Bauform Markierungen angebracht sein, die dem medizinischen Personal vorgeben, wie weit der Katheter im ersten Schritt in die Einführhülse und im zweiten Schritt ins Blutgefäß vorzuschieben ist. So kann das Personal sicher anhand der Markierung erkennen, dass der Katheter sich beispielsweise bereits im Blutgefäß befindet.

Der eingeführte Katheter liegt nach Ablösen der Einführhülse frei und das erfindungsgemäße Katheterpunktionsbesteck geteilt in Einführhülse einerseits und Sicherheitsgehäuse mitsamt Punktionsnadel und Blutauffangbehälter andererseits kann einfach und ohne ein Verletzungsrisiko entsorgt werden.

In einer vorteilhaften Ausführungsform der Erfindung wir der Katheter bis zu seinem Einschieben in das punktierte Gefäß an einer zusätzlich am Gehäuse angeordneten Halterung lösbar fixiert. Es ist hierbei zweckmäßigerweise eine Klemmhalterung vorgesehen, die einhändig zu lösen ist. So ist zum einen sichergestellt, dass bei der eigentlichen Punktierung der flexible Katheter nicht hinderlich ist oder aus dem Punktionsbesteck herausgezogen wird. Zum anderen kann dieser aber nach erfolgter Punktierung leicht aus der Fixierung gelöst und so in das punktierte Gefäß eingeschoben werden.

Eine weitere vorteilhafte Ausgestaltung der Erfindung sieht zwei in etwa parallel verlaufende Sollbruchstellen vor, die sich entlang des Abzweigabschnitts bis zur Spitze des Gehäuses bzw. der Einführhülse erstrecken. Es ist hierbei vorteilhaft, dass deren Abstand zueinander die Breite eines vom Gehäuse entfernbaren Öffnungsstreifens definiert, wobei diese Breite in etwa der des freizulegenden Katheters entspricht.

So ist sichergestellt, dass das Abziehen des Öffnungsstreifens eine schlitzförmige Öffnung über die gesamte Führungslänge des Katheters im Gehäuse freilegt, so dass der Katheter ohne Kraftaufwand aus dem Gehäuse gelöst werden kann. Dies ist eine wesentliche Verbesserung insofern, dass auf diese Weise sicher verhindert werden kann, dass beim Ablösen des Gehäuses vom in das Gefäß des Patienten eingeschobenen Katheter dieser unbeabsichtigt wieder aus dem Gefäß herausgezogen wird.

Für die Entfernung dieses Öffnungsstreifens ist weiterhin in einer zweckmäßigen Bauform eine daran im Bereich des Abzweigabschnitts angeordnete Aufreißvorrichtung vorgesehen. Dies ist insofern vorteilhaft, da so die Öffnung des Gehäuses entlang der Sollbruchstellen hin zum punktierten Patienten erfolgt und nicht von diesem weg. So wird wiederum sichergestellt, dass der eingeführte Katheter nicht unbeabsichtigt aus dem Gefäß gezogen wird, da die Zugbewegung am Gehäuse zur Entfernung des Öffnungsstreifens hin zum Patienten erfolgt.

Es ist hierbei bei einer vorteilhaften Ausgestaltung des Katheterpunktionsbestecks eine Aufreißvorrichtung an der Sollbruchstelle vorgesehen, die als zumindest einseitige Greiffläche zur besseren Zugkrafteinleitung in die Sollbruchstelle ausgebildet ist. Durch Ergreifen dieser vorsprungartigen Greiffläche, die in ihrer Form und Größe auf ein sicheres Halten beispielsweise zwischen Zeigefinger und Daumen hin gestaltet ist, kann die benötigte Kraft zur Überwindung des Bruchwiderstandes der beispielsweise gekerbt ausgebildeten Sollbruchstelle sicher eingeleitet werden.

In den Figuren 1 bis 4 wird zur näheren Beschreibung eine erfindungsgemäße Ausführungsform des einteiligen Katheterpunktionsbestecks ausführlich dargestellt.

Es zeigen
- Figur 1: eine perspektivische Darstellung des erfindungsgemäßen Katheterpunktionsbestecks etwa in Originalgröße,
- Figur 2: eine Ausschnittsvergrößerung einer perspektivischen Ansicht vor der Punktion mit eingeschobener Punktionsnadel,
- Figur 3: eine perspektivische Ansicht, bei der das Sicherheitsgehäuse 1 vom rohrartigen Gehäuse 3 abgetrennt ist mit in das Sicherheitsgehäuse 1 zurückgeführter Punktionsnadel 9,
- Figur 4: eine Draufsicht auf das vom rohrartigen Gehäuse 3 abgelöste Sicherheitsgehäuse 1.

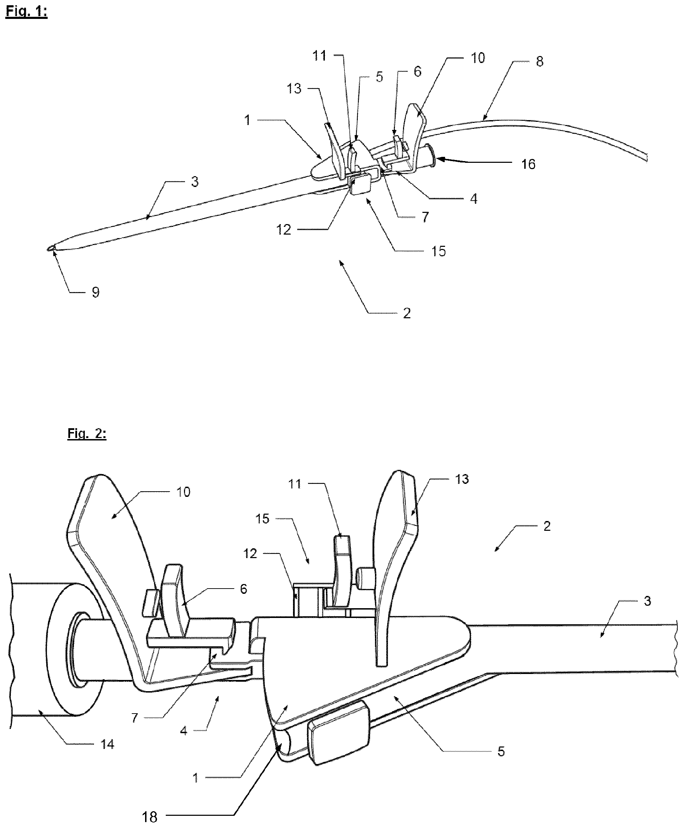

Figur 1 zeigt das erfindungsgemäße Katheterpunktionsbesteck 2 in einer Darstellung vor der Punktion eines Gefäßes. Es ist in dieser Figur kein Blutauffangbehälter 14 auf den entsprechenden Verbindungsbereich 16 an der Punktionsnadel 9 aufgesteckt.

Die Punktionsnadel 9 ist hierbei vollständig in das rohrartige Gehäuse eingeführt, sodass die scharfkantige Nadelspitze der Punktionsnadel 9 aus der vorderen Öffnung im rohrartigen Gehäuse 3 austritt. Es sind in dieser zusammengesteckten Fassung wie auch in Figur 2 zwei Arretierungsbereiche 4 und 15 zu erkennen. Der Arretierungsbereich 4 wird gebildet aus dem beweglichen Arretierungsarm 6 und einer Nadelführung 10, wobei der bewegliche Arretierungsarm 6 an einem Befestigungsabschnitt 7 angreift, der bei dieser Bauform Teil des Sicherheitsgehäuses 1 ist. Diese Arretierung 4 dient zur Fixierung der Punktionsnadel 9 im Sicherheitsgehäuse 1 vor und während der Punktion eines Gefäßes.

Nach der Punktion des Gefäßes werden die beiden Bauteile des beweglichen Arretierungsarms 6 und der Nadelführung 10 aneinander gedrückt, wodurch der bewegliche Arretierungsarm 6 angehoben und vom Befestigungsabschnitt 7 getrennt wird. Nun kann die Punktionsnadel 9 an der Nadelführung 10 gegriffen und aus dem rohrartigen Gehäuse 3 zurückgezogen werden bis in eine Arretierungsposition im Sicherheitsgehäuse 1, in der die Spitze der Punktionsnadel 9 im Sicherheitsgehäuse 1 einrastet und somit eine Verletzungsgefahr für das medizinische Personal nicht mehr erfolgen kann.

Der zweite Arretierungsabschnitt 15 betrifft die Verbindung des rohrartigen Gehäuses 3 mit dem Sicherheitsgehäuse 1. Auch diese Arretierung 15 wird in der vorliegenden Bauform gebildet aus einem Arretierungsarm 11 und einer Halteplatte 13, wobei der Arretierungsarm 11 in einer Verbindungsposition der Bauteile in eine Aufnahme 12 eingreift, die in der dargestellten beispielhaften Bauform Teil des rohrartigen Gehäuses 3 ist. Es ist hierbei allerdings variabel, ob der Arretierungsarm 11 Teil des rohrartigen Gehäuses 3 ist und die Befestigung am Sicherheitsgehäuse 1 erfolgt oder umgekehrt. Entscheidend ist, dass hier eine lösbare Arretierung erfolgt, die beispielsweise durch das Zusammendrücken von Arretierungsarm 11 und Halteplatte 13 gelöst werden kann.

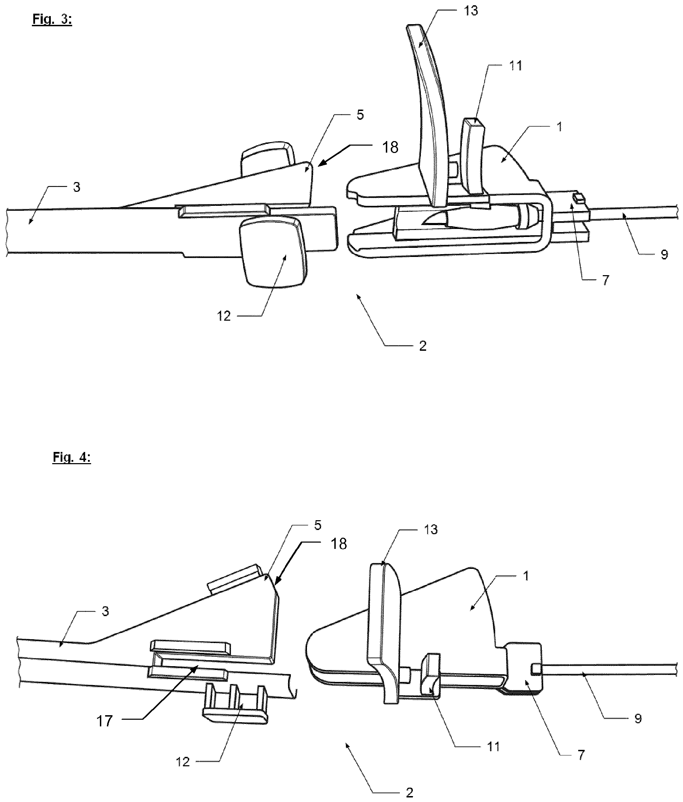

In den Figuren 3 und 4 sind die getrennten Bauelemente des rohrartigen Gehäuses 3 und Sicherheitsgehäuses 1 erkennbar. Insbesondere in Figur 4 ist dargestellt, dass das Sicherheitsgehäuse 1 in eine entsprechende Aufnahmeöffnung 17 des rohrartigen Gehäuses 3 einzuschieben ist, bis der bewegliche Arretierungsarm 11 in die Aufnahme 12 einrastet und somit beide Bauteile sicher miteinander verbunden sind.

In Figur 3 ist zudem erkennbar, dass hier die Punktionsnadel 9 bereits bis in ihre Arretierungsposition im Sicherheitsgehäuse 1 zurückgeführt worden ist. In dieser Position ist bereits im Abzweigungsabschnitt 5 der Katheter 8 in das rohrartige Gehäuse 3 bzw. das punktierte Gefäß vorgeschoben worden und gleichzeitig die Punktionsnadel 9 durch das rohrartige Gehäuse 3 zurückgeführt worden. Der Verlauf des Katheters 8 durch den Abzweigungsabschnitt 5 ist hier lediglich in Figur 1 erkennbar, wobei hier der Katheter 8 noch nicht ins rohrartige Gehäuse 3 eingeschoben ist, da hier noch die Punktionsnadel 9 sich im rohrartigen Gehäuse 3 befindet. Eine parallele Führung beider Bauteile ist im rohrartigen Gehäuse 3 bewusst nicht vorgesehen.

Nach dem Zurückziehen der Punktionsnadel 9 aus dem rohrartigen Gehäuse 3 wird der Katheter 8 vorgeschoben und kann so sauber in das Gefäß eingeführt werden. Hierbei ist ein entscheidender Vorteil der vorliegenden erfinderischen Lösung, dass beim weiteren Vorschieben des Katheters 8 in das punktierte Gefäß das Sicherheitsgehäuse 1 mit der darin gesicherten Punktionsnadel 9 und dem daran hängenden Blutauffangbehälter 14 vom rohrartigen Gehäuse 3 bereits abgenommen und zur Seite gelegt werden konnte. Es hat sich in der Praxis herausgestellt, dass das Einschieben des Katheters 8 durch das Katherterpunktionsbesteck 2 durch die noch am Katheterpunktionsbesteck 2 befindliche Punktionsnadel 9 mit Blutauffangbehälter 14 behindert wird und es so häufiger zu Fehlern bei der Legung des Katheters 8 kommt.

In der vorliegenden erfinderischen Lösung muss der Katheter 8 lediglich beim Herausziehen der Punktionsnadel 9 aus dem rohrartigen Gehäuse 3 etwas vorgeschoben werden, sodass das rohrartige Gehäuse 3 mit dem Katheter 8 gefüllt ist und somit das aufsteigende Blut in den Katheter 8 dringt. Bereits dann kann durch die entsprechende Befestigungsorgane das Sicherheitsgehäuse 1 von dem rohrartigen Gehäuse 3 abgenommen werden, wodurch das weitere Vorschieben des Katheters 8 durch diese Bauteile nicht weiter behindert wird.

In Figur 2 ist der seitliche Blick auf das rohrartige Gehäuse 3 erkennbar, wobei die Katheteraufnahme 18 für den Katheter 8 im Abzweigungsabschnitt 5 erkennbar ist. Es ist hierbei für die erfinderische Lösung relevant, dass hier eine Öffnung dieses rohrartigen Gehäuses 3 über zumindest eine zeichnerisch nicht dargestellte in Längserstreckung verlaufende Sollbruchstelle in Form eines Peel-off-Streifens möglich ist, sodass nach gelegtem Katheter 8 das rohrartige Gehäuse 3 durch diese entsprechende Sollbruchstelle geöffnet und vom Katheter 8 abgezogen werden kann ohne den gelegten Katheter 8 unbeabsichtigt aus dem punktierten Gefäß zu ziehen.

## Patentansprüche

1. Katheterpunktionsbesteck (2) umfassend ein rohrartiges Gehäuse (3) mit einem länglichen Gehäuseabschnitt, der in einen Verlängerungsabschnitt zur Führung der Punktionsnadel (9) mit Blutauffangbehälter (14), Anschlag und Sperrelement übergeht, und von dem unter einem Winkel ein Abzweigabschnitt (5) abzweigt, über welchen ein Katheter (8) in den länglichen Gehäuseabschnitt einschiebbar ist, wobei ein Sicherheitsgehäuse (1) lösbar mit dem rohrartigen Gehäuse (3) verbunden ist, wobei an oder in diesem Sicherheitsgehäuse die Punktionsnadel (9) geführt ist und (1) eine Arretierung für die nach der Punktion zurückgeführte Punktionsnadel (9) mit Blutauffangbehälter (14) vorgesehen ist, so dass nach der Punktion, dem Rückführen der Punktionsnadel (9) in des Sicherheitsgehäuse (1) und dem Vorschieben des Katheters (8) in das rohrartige Gehäuse (3) das Sicherheitsgehäuse (1) mit darin arretierter zurückgeführter Punktionsnadel (9) mit aufgesetztem Blutauffangbehälter (14) vom rohrartige Gehäuse (3) abnehmbar ist,
**dadurch gekennzeichnet dass**
- die Punktionsnadel (9) durch eine Arretierung (4) am Sicherheitsgehäuse (1) oder dem rohrartigen Gehäuse (3) in ihrer eingeschobenen Punktionsposition lösbar fixiert ist, wobei
- die Arretierung (4) der Punktionsnadel (9) mittels eines beweglichen Arretierungsarmes (6) erfolgt, der in eine Aufnahme eingreift oder an einen Befestigungsabschnitt (7) am Sicherheitsgehäuse (1) oder am rohrartigen Gehäuse (3) angreift,
- wobei die Lösung dieser Arretierung (4) durch ein Heranführen des Arretierungsarmes (6) an eine griffartige Nadelführung (10) durch ein Zusammendrücken dieser Bauteile erfolgt und
- das Sicherheitsgehäuse (1) mit dem rohrartigen Gehäuse (3) lösbar verbunden ist mittels eines weiteren beweglichen Arretierungsarmes (11), der am Sicherheitsgehäuse (1) oder am rohrartigen Gehäuse (3) angeordnet ist und in eine Aufnahme (12) am rohrartigen Gehäuse (3) oder am Sicherheitsgehäuse (1) eingreift oder an einem Befestigungsabschnitt am rohrartigen Gehäuse (3) oder am Sicherheitsgehäuse (1) angreift,
- wobei die Lösung dieser Verbindung durch ein Heranführen des Arretierungsarmes (11) an eine griffartige Halteplatte (13) am Sicherheitsgehäuse (1) oder am rohrartigen Gehäuse (3) durch ein Zusammendrücken dieser Bauteile erfolgt.

2. Katheterpunktionsbesteck (2) nach Anspruch 1,
**dadurch gekennzeichnet, dass**
- das rohrartige Gehäuse (3) des Katheterpunktionsbesteck (2) einteilig und geschlossen ausgebildet ist,
- wobei der längliche, rohrförmig geschlossene Gehäuseabschnitt (3) sowohl die mit ihrer Spitze aus dem länglichen Gehäuseabschnitt vorderseitig austretende, sich längs des Gehäuses (3) erstreckende Punktionsnadel (9) führt, als auch gleichzeitig den Führungskanal für den Katheter (8) bildet, der nach erfolgter Punktion und Rückführung der Punktionsnadel (9) bis hinter die Abzweigstelle des Abzweigabschnitts (5) über diesen Abzweigabschnitt (5) in den länglichen Gehäuseabschnitt eingeführt wird,
- wobei entlang des Abzweigabschnitts (5) bis zur Spitze des länglichen Gehäuseabschnitts (3) zumindest eine Sollbruchstelle verläuft, mithilfe derer das rohrartige Gehäuse (3) des Katheterpunktionsbestecks (2), nachdem der Katheter (8) hindurch geschoben wurde, aufgebrochen werden kann, um das Gehäuse (3) vom Katheter (8) zu entfernen.

3. Katheterpunktionsbesteck nach einem der vorangegangenen Ansprüche 1 und 2,
**dadurch gekennzeichnet, dass**
am Gehäuse (3) mindestens eine Halterung angeordnet ist, die den Katheter (8) bis zur Einschiebung desselben in das Gehäuse (3) im Abzweigabschnitt (5) lösbar fixiert.

4. Katheterpunktionsbesteck nach einem der vorangegangenen Ansprüche,
**dadurch gekennzeichnet, dass**
die Spitze des länglichen Gehäuseabschnitts(3), aus welchem die Punktionsnadel (9) zur Punktion austritt, spitz gerundet ausgebildet ist.

5. Katheterpunktionsbesteck nach einem der Ansprüche einem der vorangegangenen Ansprüche,
**dadurch gekennzeichnet, dass**
ein Anschlagpunkt im Sicherheitsgehäuse (1) für die zurückführbare Punktionsnadel (9) nach erfolgter Punktion definiert ist, wobei ein Sperrelement zum Verhindern eines erneuten Einschiebens der Punktionsnadel (9) in das Gehäuse (3) im lösbaren Sicherheitsgehäuse angeordnet ist.

6. Katheterpunktionsbesteck nach Anspruch 5,
**dadurch gekennzeichnet, dass**
das Sperrelement als Spreizkörper im vorderen Abschnitt der Punktionsnadel (9) ausgebildet ist, dessen Fangarme beim Zurückziehen der Punktionsnadel (9) über den Abzweigabschnitt (5) hinaus in eine Absatzstufe im Sicherheitsgehäuse (1) einspreitzen und die Punktionsnadel (9) so arretieren.

7. Katheterpunktionsbesteck nach einem der vorangegangenen Ansprüche 2 bis 6,
**dadurch gekennzeichnet, dass**
die mindestens eine Sollbruchstelle mit einer daran angeordneten Aufreißvorrichtung versehen ist.

8. Katheterpunktionsbesteck nach Anspruch 7,
**dadurch gekennzeichnet, dass**
die Aufreißvorrichtung an der Sollbruchstelle als zumindest eine Greiffläche zur besseren Zugkrafteinleitung in die zumindest eine Sollbruchstelle ausgebildet ist.

9. Katheterpunktionsbesteck nach einem der vorangegangenen Ansprüche 2 bis 8,
**dadurch gekennzeichnet, dass**
zwei in etwa parallel verlaufende Sollbruchstellen sich entlang des Abzweigabschnitts (5) bis zur Spitze des Gehäuses (3) erstrecken, deren Abstand zueinander die Breite eines vom Gehäuse (3) entfernbaren Öffnungsstreifens definiert, wobei diese Breite in etwa der des freizulegenden Katheters (8) entspricht.

10. Katheterpunktionsbesteck nach einem der vorangegangenen Ansprüche,
**dadurch gekennzeichnet, dass**
am Katheter (8) Markierungen angebracht sind, die in Relation zu einem Bezugspunkt am Katheterpunktionsbesteck (2) anzeigen, wie weit der Katheter (8) in einem oder mehreren Schritten in die Einführhülse des rohrartige Gehäuse (3) bzw. ins Blutgefäß vorgeschoben ist.

## Claims

1. Catheter puncture set (2) comprising a tubular housing (3) having an elongate housing portion which merges into an extension portion for guiding the puncture needle (9), with a blood-collection container (14), stop and blocking element, and from which a branch portion (5) branches off at an angle, it being possible for a catheter (8) to be pushed into the elongate housing portion via the branch portion, wherein a safety housing (1) is connected in a releasable manner to the tubular housing (3), wherein the puncture needle (9) is guided on or in this safety housing and (1) an arresting means is provided for the puncture needle (9) with blood-collection container (14), the puncture needle being retracted following the puncturing step, and therefore, following the puncturing step, the retraction of the puncture needle (9) into the safety housing (1) and the advancement of the catheter (8) into the tubular housing (3), the safety housing (1) along with the retracted puncture needle (9), with blood-collection container (14) attached, arrested therein can be removed from the tubular housing (3),
**characterized in that**
- an arresting means (4) fixes the puncture needle (9) in a releasable manner in its pushed-in puncturing position on the safety housing (1) or the tubular housing (3), wherein
- the puncture needle (9) is arrested (4) by means of a movable arresting arm (6), which engages in a mount or acts on a fastening portion (7) on the safety housing (1) or on the tubular housing (3),
- wherein this arresting means (4) is released by virtue of the arresting arm (6) being guided up to a handle-like needle guide (10) as a result of these components being pressed together, and
- the safety housing (1) is connected in a releasable manner to the tubular housing (3) by means of a further movable arresting arm (11) which is arranged on the safety housing (1) or on the tubular housing (3) and which engages in a mount (12) on the tubular housing (3) or on the safety housing (1) or acts on a fastening portion on the tubular housing (3) or on the safety housing (1),
- wherein this connection is released by virtue of the arresting arm (11) being guided up to a handle-like retaining plate (13) on the safety housing (1) or on the tubular housing (3) as a result of these components being pressed together.

2. Catheter puncture set (2) according to Claim 1, **characterized in that**
- the tubular housing (3) of the catheter puncture set (2) is of a single-piece and closed design,
- the elongate housing portion (3), closed to form a tube, both guides the puncture needle (9), which has its point exiting from the front side of the elongate housing portion and extends along the housing (3), and, at the same time, forms the guide channel for the catheter (8), which, once puncturing has taken place and the puncture needle (9) has been retracted to behind the branching-off location of the branch portion (5), is introduced into the elongate housing portion via said branch portion (5),
- wherein at least one predetermined breaking location runs along the branch portion (5) as far as the tip of the elongate housing portion (3), said predetermined breaking location enabling the tubular housing (3) of the catheter puncture set (2), once the catheter (8) has been pushed through, to be broken open, in order for the housing (3) to be removed from the catheter (8).

3. Catheter puncture set according to either of the preceding Claims 1 and 2, **characterized in that** the housing (3) has arranged on it at least one holder, which fixes the catheter (8) in a releasable manner in the branch portion (5) until it is pushed into the housing (3).

4. Catheter puncture set according to one of the preceding claims, **characterized in that** the tip of the elongate housing portion (3), from which the puncture needle (9) exits for puncturing purposes, is rounded.

5. Catheter puncture set according to one of the preceding claims, **characterized in that** a stop point in the safety housing (1) for the retractable puncture needle (9) is defined once puncturing has taken place, wherein a blocking element is arranged in the releasable safety housing in order to prevent the puncture needle (9) from being pushed into the housing (3) anew.

6. Catheter puncture set according to Claim 5, **characterized in that** the blocking element is designed in the form of an expansion body in the front portion of the puncture needle (9), the intercepting arms of said body expanding into a step in the safety housing (1) and thus arresting the puncture needle (9), when the puncture needle (9) is drawn back beyond the branch portion (5).

7. Catheter puncture set according to one of the preceding Claims 2 to 6, **characterized in that** the at least one predetermined breaking location is provided with a tearing-open device arranged thereon.

8. Catheter puncture set according to Claim 7, **characterized in that** the tearing-open device at the predetermined breaking location is designed in the form of at least one gripping surface for the improved introduction of tensile forces into the at least one predetermined breaking location.

9. Catheter puncture set according to one of the preceding Claims 2 to 8, **characterized in that** two approximately parallel predetermined breaking locations extend along the branch portion (5) as far as the tip of the housing (3), the distance between said predetermined breaking locations defining the width of an opening strip which can be removed from the housing (3), wherein said width corresponds approximately to that of the catheter (8) which is to be uncovered.

10. Catheter puncture set according to one of the preceding claims, **characterized in that** the catheter (8) has applied to it markings which indicate, in relation to a reference point on the catheter puncture set (2), how far the catheter (8) has been advanced, in one or more steps, into the introduction sleeve of the tubular housing (3) or into the blood vessel.

## Revendications

1. Kit de ponction à cathéter (2) comprenant un boîtier de type tube (3) ayant une portion de boîtier allongée qui se prolonge par une portion de prolongement pour le guidage de l'aiguille de ponction (9) comprenant un récipient de collecte de sang (14), une butée et un élément d'arrêt, et de laquelle part, suivant un certain angle, une portion de ramification (5) par le biais de laquelle un cathéter (8) peut être introduit dans la portion de boîtier allongée, un boîtier de sécurité (1) étant relié de manière amovible au boîtier de type tube (3), l'aiguille de ponction (9) étant guidée sur ou dans ce boîtier de sécurité (1) et un arrêt étant prévu pour l'aiguille de ponction (9) ramenée après la ponction avec le récipient de collecte de sang (14), de telle sorte qu'après la ponction, le retour de l'aiguille de ponction (9) dans le boîtier de sécurité (1) et l'avance du cathéter (8) dans le boîtier de type tube (3), le boîtier de sécurité (1), avec l'aiguille de ponction (9) arrêtée ramenée dans celui-ci, puisse être enlevé avec le récipient de collecte de sang (14) hors du boîtier de type tube (3),
**caractérisé en ce que**
- l'aiguille de ponction (9) est fixée de manière amovible dans sa position de ponction enfoncée par un arrêt (4) au niveau du boîtier de sécurité (1) ou au niveau du boîtier de type tube (3),
- l'arrêt (4) de l'aiguille de ponction (9) s'effectuant au moyen d'un bras d'arrêt mobile (6) qui s'engage dans un logement ou qui vient en prise au niveau d'une portion de fixation (7) sur le boîtier de sécurité (1) ou sur le boîtier de type tube (3),
- le desserrage de cet arrêt (4) s'effectuant par le rapprochement du bras d'arrêt (6) d'un guide d'aiguille de type prise (10) par compression de ces composants et
- le boîtier de sécurité (1) étant connecté de manière amovible au boîtier de type tube (3) au moyen d'un bras d'arrêt mobile supplémentaire (11) qui est disposé au niveau du boîtier de sécurité (1) ou au niveau du boîtier de type tube (3) et qui s'engage dans un logement (12) au niveau du boîtier de type tube (3) ou au niveau du boîtier de sécurité (1) ou qui vient en prise au niveau d'une portion de fixation sur le boîtier de type tube (3) ou sur le boîtier de sécurité (1),
- le desserrage de cette connexion s'effectuant par un rapprochement du bras d'arrêt (11) d'une plaque de retenue de type prise (13) au niveau du boîtier de sécurité (1) ou au niveau du boîtier de type tube (3) par compression de ces composants.

2. Kit de ponction à cathéter (2) selon la revendication 1,
**caractérisé en ce que**
- le boîtier de type tube (3) du kit de ponction à cathéter (2) est réalisé sous forme fermée et d'une seule pièce,
- la portion de boîtier allongée (3), fermée sous forme tubulaire, guidant l'aiguille de ponction (9) s'étendant le long du boîtier (3) et sortant avec sa pointe hors de la portion de boîtier allongée, et formant en même temps également le canal de guidage pour le cathéter (8) qui, après la ponction et le retour de l'aiguille de ponction (9) jusque derrière la zone de ramification de la portion de ramification (5), est introduit par le biais de cette portion de ramification (5) dans la portion de boîtier allongée,
- au moins une zone destinée à la rupture s'étendant le long de la portion de ramification (5) jusqu'à la pointe de la portion de boîtier allongée (3), à l'aide de laquelle le boîtier de type tube (3) du kit de ponction à cathéter (2), après que le cathéter (8) a été enfoncé à travers celui-ci, peut être rompu afin d'enlever le boîtier (3) du cathéter (8) .

3. Kit de ponction à cathéter selon l'une quelconque des revendications précédentes 1 et 2,
**caractérisé en ce**
**qu'**au moins une fixation est disposée au niveau du boîtier (3), laquelle fixe de manière amovible le cathéter (8) jusqu'à son enfoncement dans le boîtier (3) dans la portion de ramification (5).

4. Kit de ponction à cathéter selon l'une quelconque des revendications précédentes,
**caractérisé en ce que**
la pointe de la portion de boîtier allongée (3) de laquelle sort l'aiguille de ponction (9) pour la ponction est réalisée sous forme de pointe arrondie.

5. Kit de ponction à cathéter selon l'une quelconque des revendications précédentes,
**caractérisé en ce**
**qu'**un point de butée est défini dans le boîtier de sécurité (1) pour l'aiguille de ponction (9) pouvant être ramenée une fois la ponction terminée, un élément de blocage pour empêcher une réinsertion de l'aiguille de ponction (9) dans le boîtier (3) étant disposé dans le boîtier de sécurité amovible.

6. Kit de ponction à cathéter selon la revendication 5,
**caractérisé en ce que**
l'élément de blocage est réalisé sous forme de corps d'écartement dans la portion avant de l'aiguille de ponction (9), dont les bras de capture, lors du retour de l'aiguille de ponction (9), s'écartent au-delà de la portion de ramification (5) dans un épaulement en gradin dans le boîtier de sécurité (1) et arrêtent ainsi l'aiguille de ponction (9).

7. Kit de ponction à cathéter selon l'une quelconque des revendications précédentes 2 à 6,
**caractérisé en ce que**
l'au moins une zone destinée à la rupture est pourvue d'un dispositif d'arrachage disposé sur celle-ci.

8. Kit de ponction à cathéter selon la revendication 7,
**caractérisé en ce que**
le dispositif d'arrachage au niveau de la zone destinée à la rupture est réalisé sous forme d'au moins une surface de préhension pour une meilleure introduction de la force de traction dans l'au moins une zone destinée à la rupture.

9. Kit de ponction à cathéter selon l'une quelconque des revendications précédentes 2 à 8,
**caractérisé en ce que**
deux zones destinées à la rupture s'étendant approximativement parallèlement s'étendent le long de la portion de ramification (5) jusqu'à la pointe du boîtier (3), leur espacement l'une de l'autre définissant la largeur d'une bande d'ouverture pouvant être enlevée du boîtier (3), cette largeur correspondant approximativement à celle du cathéter (8) à exposer.

10. Kit de ponction à cathéter selon l'une quelconque des revendications précédentes,
**caractérisé en ce que**
des marquages sont réalisés sur le cathéter (8), lesquels, en relation avec un point de référence sur le kit de ponction à cathéter (2), indiquent sur quelle distance le cathéter (8) est avancé en une ou plusieurs étapes dans la douille d'insertion du boîtier de type tube (3) ou dans le vaisseau sanguin.
